(19) Europäisches Patentamt / European Patent Office / Office européen des brevets

(11) **EP 4 316 363 A1**

(12) **DEMANDE DE BREVET EUROPEEN**

(43) Date de publication:
**07.02.2024 Bulletin 2024/06**

(21) Numéro de dépôt: **23188547.6**

(22) Date de dépôt: **28.07.2023**

(51) Classification Internationale des Brevets (IPC):
**A61B 5/0225** (2006.01) **A61B 5/0285** (2006.01)
**A61B 5/02** (2006.01)

(52) Classification Coopérative des Brevets (CPC):
**A61B 5/0225; A61B 5/0285;** A61B 5/02007;
A61B 2560/0223

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**KH MA MD TN**

(30) Priorité: **31.07.2022 FR 2207947**

(71) Demandeur: **Commissariat à l'énergie atomique et aux énergies alternatives**
**75015 Paris (FR)**

(72) Inventeurs:
• **BONNET, Stéphane**
**38054 Grenoble cedex 09 (FR)**
• **BEDNAREK, Xavier**
**38054 Grenoble cedex 09 (FR)**

(74) Mandataire: **INNOV-GROUP**
**310, avenue Berthelot**
**69372 Lyon Cedex 08 (FR)**

(54) **DISPOSITIF ET PROCÉDÉ DE MESURE D'UNE PRESSION ARTÉRIELLE**

(57) Procédé de détermination d'une pression artérielle d'un utilisateur, en mesurant un paramètre physiologique, le paramètre physiologique passant par un extremum lorsque la pression transmurale de l'artère est nulle, le procédé comportant:

- a) application d'une pression sur l'artère, de façon à modifier la pression transmurale de l'artère;
- b) mesure du paramètre physiologique de l'utilisateur à l'aide d'un capteur;
- c) établissement d'une fonction de calibration, la fonction de calibration déterminant une relation entre la pression transmurale et le paramètre;
- d) application d'une pression sur l'artère, à un instant de mesure et mesure du paramètre physiologique à l'instant de mesure;
- e) estimation d'une pression transmurale à l'instant de mesure;
- f) à partir de la pression transmurale estimée lors de l'étape e) et de la pression appliquée à l'instant de mesure, estimation d'une pression artérielle de l'utilisateur.

Fig. 4

EP 4 316 363 A1

**Description**

## DOMAINE TECHNIQUE

Le domaine technique de l'invention est la mesure de la pression artérielle.

## ART ANTERIEUR

La plupart des dispositifs permettant de mesurer la pression artérielle utilisent un capteur de pression couplé à un brassard de compression disposé sur un membre, généralement un bras. La caractérisation de la pression artérielle est effectuée en mesurant la pression exercée par le brassard en un ou plusieurs instants caractéristiques. Le capteur de pression est sensible aux battements cardiaques et à leur amplitude. En général, les dispositifs sont disposés autour de l'artère brachiale.

Dans les tensiomètres grand public, un capteur de pression détermine la pression d'air dans le brassard. La compression du brassard est effectuée de manière à obtenir une occlusion artérielle. Lors de la déflation ou de l'inflation du brassard, des oscillations de pression apparaissent. Les oscillations augmentent jusqu'à atteindre, transitoirement, une amplitude maximale. A cet instant la pression dans le brassard est considérée égale à la pression artérielle moyenne dans l'artère brachiale. A partir de l'amplitude maximale détectée, les instants correspondant respectivement aux pressions artérielles systolique et diastolique sont estimés sur la base de lois empiriques. Il est usuellement considéré que :

- l'instant correspondant à la pression artérielle systolique est antérieur à l'instant de la pression artérielle moyenne, et correspond à une amplitude d'oscillation d'un certain pourcentage de l'amplitude maximale, par exemple 55% ;
- l'instant correspondant à la pression artérielle diastolique est postérieur à l'instant de la pression artérielle moyenne, et correspond à une amplitude d'oscillation d'un autre pourcentage de l'amplitude maximale, par exemple 85%.

La pression artérielle moyenne est donc une grandeur pouvant être aisément mesurée par un dispositif grand public. A partir ce cette mesure, la pression artérielle systolique et la pression artérielle diastolique peuvent être calculées. Il est courant que la pression artérielle moyenne, bien que mesurée, ne soit pas communiquée à l'utilisateur, au profit des pressions artérielles systoliques ou diastoliques.

La pression transmurale d'une artère correspond à une différence entre la pression interne à l'artère, ou pression artérielle, et la pression appliquée sur l'artère par un moyen d'application de pression sur une artère, comme un brassard.

$$P_t = P - P_{ext} \quad (1)$$

Lorsque la pression appliquée par le brassard correspond à la pression artérielle moyenne, la pression transmurale est considérée nulle. L'artère atteint alors son maximum de compliance. La compliance d'une artère correspond au ratio de la variation de son aire de section sur la variation de la pression transmurale.

Ainsi :

$$C(p_t) = \frac{\Delta A}{\Delta p_t} \quad (2)$$

où :

- $p_t$ est la pression transmurale ;
- $C(p_t)$ est la compliance de l'artère ;
- $\Delta A$ est la variation de l'aire de section l'artère ;
- $\Delta p_t$ est la variation de la pression transmurale.

L'aire de section est le volume de sang présent dans une section d'artère de longueur L, divisé par ladite longueur L.

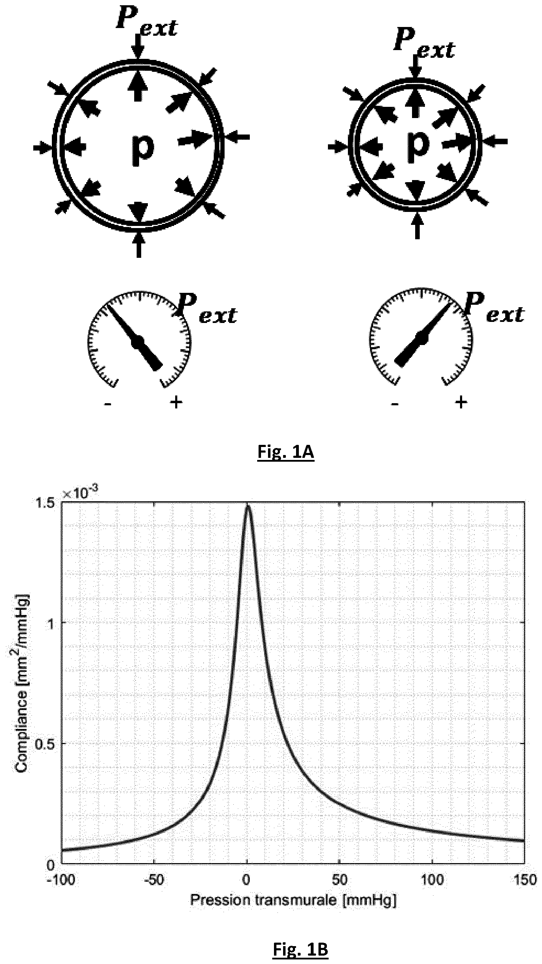

La figure 1A schématise une application d'une pression autour d'une artère. Pour une pression artérielle donnée, plus la pression appliquée sur l'artère augmente, plus l'aire de section de l'artère diminue.

La figure 1B montre l'évolution de la compliance de l'artère (axe des ordonnées - unité mm$^2$ par mm de mercure) en fonction de la pression transmurale (axe des abscisses - unité mm de mercure). On observe que la compliance de l'artère, c'est-à-dire son élasticité, est maximale lorsque la pression transmurale est nulle, c'est-à-dire lorsque la pression à l'extérieur de l'artère correspond à la pression artérielle moyenne. Cette propriété est exploitée dans les dispositifs de mesure de la pression artérielle moyenne précédemment décrits.

Dans l'art antérieur, certains développements ont été effectués, de façon à estimer une pression artérielle à partir d'une mesure d'un paramètre physiologique pouvant être mesuré avec un dispositif plus compact que les brassards équipés de capteurs de pression. Le paramètre physiologique dépend de la compliance de l'artère. Il peut s'agir par exemple d'une amplitude d'oscillation ou une durée de transit d'une onde de pouls entre deux détecteurs ou une vitesse d'onde de pouls. Un tel paramètre est aisément mesurable, par exemple par le biais de mesures optiques de type photopléthysmographie (PPG). Cependant, selon les procédés existants, l'estimation de la pression artérielle à partir de mesures du paramètre physiologique suppose une phase de calibration, au cours de laquelle on effectue des mesures du paramètre physiologique pour différentes pressions artérielles d'un utilisateur. Cependant, ce type de calibration suppose que la pression artérielle de l'utilisateur puisse varier. Une telle calibration est donc consommatrice de temps, et n'est valable que dans la plage des pressions artérielles de l'utilisateur durant la phase de calibration. Il est relativement aisé d'augmenter la pression artérielle d'un utilisateur, par exemple en le soumettant à un stress ou à un exercice physique. Cependant, il est plus difficile d'abaisser la tension artérielle d'un utilisateur. On comprend que l'estimation d'une pression artérielle, directement à partir de mesures d'un paramètre physiologique suppose une phase de calibration délicate, qui dépend de l'aptitude à pouvoir moduler la pression artérielle de l'utilisateur.

Le document US2010/0241011 décrit un dispositif d'estimation de la pression artérielle à partir d'un paramètre physiologique de type temps de transit de l'onde de pouls. L'estimation de la tension artérielle est basée sur un modèle analytique paramétrique, dont les paramètres sont quantifiés lors d'une phase de calibration. Le modèle analytique est basé sur une détermination de deux temps de transit différents, ce qui suppose l'application de deux niveaux de pression différents. En outre, le modèle analytique est formé d'une somme pondérée de deux fonctions exponentielles de la pression artérielle, ce qui est relativement complexe.

Le document US2017/0215749 décrit un dispositif d'estimation de la pression artérielle sur la base d'un modèle analytique. Ce dernier suppose plusieurs mesures d'un paramètre physiologique, en particulier une amplitude d'oscillation, en appliquant différents niveaux de pression à une artère.

Les inventeurs proposent une méthode permettant une estimation d'une pression artérielle à partir d'une mesure d'un paramètre physiologique d'un utilisateur, aisément mesurable. Cependant, contrairement aux procédés décrits dans le paragraphe précédent, la méthode faisant l'objet de l'invention est basée sur une calibration rapide, et plus simple à mettre en oeuvre, sans avoir à modifier, la pression artérielle de l'utilisateur. De ce fait, la calibration peut être fréquemment renouvelée. De plus, la méthode peut être mise en oeuvre, après la calibration, en appliquant qu'un seul niveau de pression autour d'une artère.

## EXPOSE DE L'INVENTION

Un premier objet de l'invention est un procédé de détermination d'une pression artérielle d'un utilisateur, en mesurant un paramètre physiologique, le paramètre physiologique passant par un extremum lorsque la pression transmurale de l'artère est nulle, le procédé comportant les étapes suivantes :

- a) application d'une pression sur l'artère, de façon à modifier la pression transmurale de l'artère, la pression transmurale correspondant à une différence entre la pression artérielle et la pression appliquée sur l'artère, la pression appliquée étant connue ou mesurée ;
- b) simultanément à l'étape a), mesure du paramètre physiologique de l'utilisateur à l'aide d'un capteur;
- les étapes a) et b) étant réitérées en différents instants de calibration, en modifiant la pression appliquée sur l'artère, de façon que lors d'une étape b), l'extremum du paramètre physiologique soit mesuré, la pression appliquée correspondant alors à une pression artérielle moyenne de l'utilisateur lors des instants de calibration;
- c) à partir de la pression appliquée et du paramètre physiologique mesuré lors des étapes a) et b), établissement d'une fonction de calibration, la fonction de calibration déterminant une relation entre la pression transmurale et le paramètre physiologique;
- d) suite aux instants de calibration, application d'une pression sur l'artère, à au moins un instant de mesure et mesure du paramètre physiologique à l'instant de mesure, la pression appliquée à l'instant de mesure étant connue ou mesurée ;
- e) application de la fonction de calibration résultant de c) au paramètre physiologique mesuré à l'instant de mesure, de façon à estimer une pression transmurale à l'instant de mesure ;
- f) à partir de la pression transmurale estimée lors de l'étape e) et de la pression appliquée à l'instant de mesure, estimation d'une pression artérielle de l'utilisateur.

L'étape c) est effectuée par une unité de traitement programmée à cet effet. L'unité de traitement peut comporter un microprocesseur.

L'étape f) peut comporter une addition de la pression transmurale estimée lors de l'étape e) et de la pression appliquée lors de l'étape d).

Le paramètre physiologique peut être un paramètre relatif à une compliance de l'artère.

Lors de l'étape a) et/ou de l'étape d), la pression peut être appliquée au moyen d'un moyen d'application d'une pression sur l'artère de l'utilisateur, par exemple un brassard gonflable, configuré pour comprimer l'artère.

La fonction de calibration peut être obtenue en appliquant un modèle de régression à partir de la pression appliquée et du paramètre physiologique mesuré à chaque instant de calibration. Selon une possibilité :

- lors des étapes a) à b), la pression appliquée sur l'artère varie jusqu'à une pression de référence à laquelle le paramètre physiologique atteint l'extremum, la pression de référence correspondant à une pression artérielle moyenne lors des instants de calibration.
- lors de l'étape d), la pression appliquée sur l'artère est inférieure à la pression de référence.

Lors de l'étape d), la pression appliquée par sur l'artère peut être inférieure à 50% ou à 25 % de la pression de référence.

Selon une possibilité :

- les étapes a) et b) sont mises en oeuvre en prenant en compte différents paramètres physiologiques, de façon à mesurer un extremum de chaque paramètre physiologique ;
- lors de l'étape c), la fonction de calibration détermine une relation entre la pression transmurale et chaque paramètre physiologique ;
- l'étape d) comporte une mesure de chaque paramètre physiologique à l'instant de mesure ;
- lors de l'étape e), la pression transmurale à l'instant de mesure est estimée à partir de la fonction de calibration déterminée lors de l'étape c) et des paramètres physiologiques mesurés lors de l'étape d).

Selon une possibilité, les étapes d) à f) sont mises en oeuvre en différents instants de mesure, selon une fréquence supérieure à une fréquence cardiaque de l'utilisateur, de façon à obtenir une évolution de la pression artérielle de l'utilisateur entre lesdits instants de mesure.

lors de l'étape f), la pression artérielle estimée peut être une pression artérielle moyenne de l'utilisateur.

Un deuxième objet de l'invention est un dispositif d'estimation d'une pression artérielle d'un utilisateur, comportant :

- un capteur, configuré pour être appliqué face à une artère de l'utilisateur, et configuré pour mesurer un paramètre physiologique de l'utilisateur, le paramètre physiologique passant par un extremum lorsque la pression transmurale de l'artère est nulle ;
- un moyen d'application d'une pression sur l'artère de l'utilisateur, par exemple un brassard gonflable, configuré pour appliquer une pression variable sur l'artère de l'utilisateur ;
- une unité de traitement, destinée à mettre en oeuvre les étapes c), e) et f) du procédé selon le premier objet de l'invention à partir du paramètre physiologique mesuré par le capteur et de la pression appliquée sur l'artère.

Le dispositif peut comporter un capteur de pression, configuré pour quantifier la pression appliquée sur l'artère par le moyen d'application d'une pression sur l'artère de l'utilisateur.

Le capteur peut être choisi parmi : un capteur acoustique, un capteur optique, un capteur tonométrique ou un capteur d'impédance ou un capteur électromécanique. Le capteur peut être maintenu autour d'un membre de l'utilisateur par un bracelet, moyen d'application d'une pression sur l'artère de l'utilisateur étant intégré dans le bracelet ou solidaire du bracelet.

L'invention sera mieux comprise à la lecture de l'exposé des exemples de réalisation présentés, dans la suite de la description, en lien avec les figures listées ci-dessous.

## FIGURES

La figure 1A illustre une variation de l'aire de section d'une artère en fonction de la pression appliquée sur l'artère.
La figure 1B représente une variation de la compliance en fonction de la pression transmurale.
Les figures 2A et 2B sont des schémas représentant le dispositif.
La figure 3 résume les principales étapes de l'invention.
La figure 4 schématise une augmentation progressive d'une pression appliquée par un brassard.

**EXPOSE DE MODES DE REALISATION PARTICULIERS**

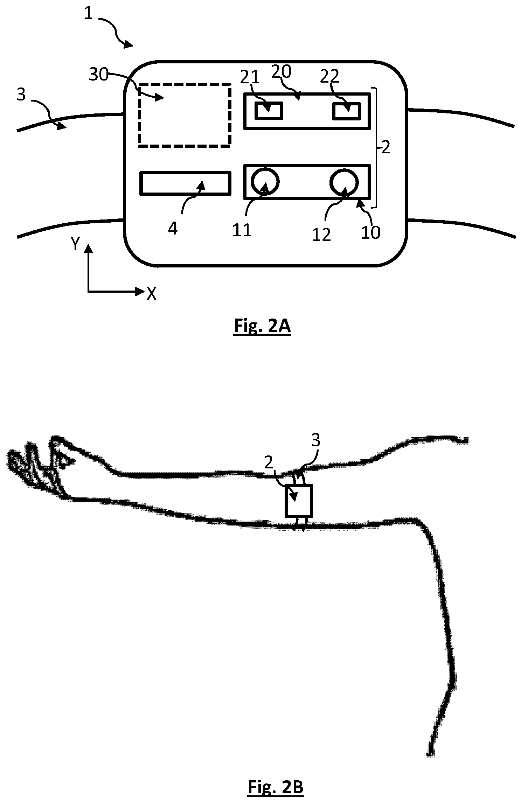

Les figures 2A et 2B schématisent un dispositif 1 selon l'invention. Le dispositif est destiné à être porté par un utilisateur, en particulier autour d'un membre, par exemple un bras ou un poignet. Le dispositif 1 comporte un bracelet 3, permettant un maintien du dispositif autour du membre de l'utilisateur. De préférence, il s'agit d'un bracelet gonflable, de type brassard. Le bracelet gonflable est configuré pour comprimer le membre lorsqu'il est gonflé. D'une façon plus générale, le dispositif comporte un moyen d'application d'une pression sur une artère de l'utilisateur.

Le dispositif comporte un capteur 2 configuré pour mesurer au moins un paramètre physiologique de l'utilisateur. Le paramètre physiologique mesuré varie en fonction d'une pression transmurale d'une artère de l'utilisateur, face à laquelle le dispositif 1 est placé. Il s'agit notamment d'un paramètre physiologique lié à la compliance de l'artère susceptible de varier en fonction de la pression transmurale de l'artère.

Le paramètre physiologique peut être, de façon non limitative, une vitesse d'onde de pouls, ou une amplitude d'oscillation de la paroi de l'artère.

Le paramètre physiologique présente une valeur extrémale, minimale ou maximale, lorsque l'artère est à son maximum de compliance, c'est-à-dire lorsque la pression appliquée sur l'artère, correspond à la pression artérielle : la pression transmurale est alors nulle. Lorsque le paramètre physiologique est la vitesse d'onde de pouls, il atteint une valeur minimale lorsque la pression transmurale est nulle. Lorsque le paramètre physiologique est l'amplitude d'oscillation, il atteint une valeur maximale lorsque la pression transmurale est nulle.

Dans l'exemple représenté, le capteur 2 comporte une source de lumière 10 et un photodétecteur 20. La source de lumière 10 comporte une première source de lumière élémentaire 11 et une deuxième source de lumière élémentaire 12. Les première et deuxième sources de lumière sont distantes l'une de l'autre, selon une direction selon laquelle s'étend l'artère. La distance entre la première et la deuxième source de lumière élémentaire peut être inférieure à 5 cm. Plus la distance est faible, plus le dispositif est compact, et peut être par exemple maintenu par un bracelet disposé autour d'un bras de l'utilisateur. Le photodétecteur 20 comporte un premier photodétecteur élémentaire 21 et un deuxième photodétecteur élémentaire 22.

La première source de lumière élémentaire 11 et la deuxième source de lumière élémentaire 12 sont par exemple des diodes électroluminescente (LED : Light Emitting Diode). La première source de lumière élémentaire 11 émet un faisceau lumineux incident se propageant vers les tissus de l'utilisateur, selon un axe de propagation, de préférence perpendiculaire à la surface du corps de l'utilisateur. Le faisceau lumineux est de préférence émis selon une bande spectrale étroite, de préférence < 50 nm, dans une plage spectrale comprise entre 400 nm et 1100 nm.

Le premier photodétecteur élémentaire 21 et le deuxième photodétecteur élémentaire 22 sont par exemple des photodiodes.

De façon connue, les photons du faisceau lumineux pénètrent dans les tissus de l'utilisateur et une partie d'entre eux est rétrodiffusée selon une direction parallèle à l'axe de propagation, dans un sens opposé à ce dernier. Les photons rétrodiffusés résultant de la première source de lumière élémentaire 11 sont détectés par le premier photodétecteur élémentaire 21. Les photons rétrodiffusés résultant de la deuxième source de lumière élémentaire 12 sont détectés par le deuxième photodétecteur élémentaire 22.

Le principe de fonctionnement du capteur 2 est basé sur le fait qu'à chaque battement cardiaque, l'afflux sanguin face à chaque photodétecteur élémentaire entraîne une modulation de l'absorption la lumière se propageant à travers les tissus biologiques formant le corps de l'utilisateur. Il en résulte une modulation de l'intensité de la lumière détectée par le premier photodétecteur élémentaire 21 et par le deuxième photodétecteur élémentaire 22. Ainsi, l'intensité détectée par chaque photodétecteur forme un signal périodique, dont la fréquence fondamentale correspond à la fréquence cardiaque. L'espacement entre les deux photodétecteurs élémentaires 21, 22 permet une détermination d'un décalage temporel entre les signaux périodiques résultant respectivement du premier photodétecteur et du deuxième photodétecteur. L'estimation du décalage temporel, appelé temps de transit de l'onde de pouls, permet une estimation d'une vitesse, dite « vitesse d'onde de pouls » (VOP) en divisant par la distance entre le premier photodétecteur et le deuxième photodétecteur.

De façon alternative, la détermination de la VOP peut être effectuée par une mesure passive, par exemple en utilisant deux capteurs mécaniques ou électromécaniques de type tonomètre, espacés d'une distance connue : avec ce type de capteur, on mesure une déformation du tissu sous l'effet de l'onde de pouls, sans signal d'excitation.

D'autres méthodes de mesure du paramètre physiologique peuvent être basées sur une excitation électrique des tissus, et d'une mesure d'une réponse, également électrique, des tissus, selon le principe de mesures d'impédance électrique. Sous l'effet d'une variation périodique du volume sanguin, l'impédance électrique varie de façon périodique. Ainsi, une mesure de l'impédance électrique permet un suivi de l'activité cardiaque. Le décalage entre les variations périodiques d'impédances électriques permet d'estimer la vitesse d'onde de pouls. Ce type de modalité est décrit dans la publication Bassem I. et al « Multi-source multi-frequency bio-impedance measurement method for localized pulse wave monitoring », 2020. La mesure d'une vitesse d'onde de pouls peut également être obtenue par une méthode acoustique, en déterminant une variation périodique de l'impédance acoustique des tissus induite par l'activité cardiaque.

Le principe est alors basé sur une émission d'une onde acoustique se propageant vers les tissus, et sur une détection d'une onde acoustique réfléchie par les tissus. Le capteur 2 peut comporter deux capteurs acoustiques élémentaires distants l'un de l'autre, la distance entre les deux capteurs étant connue. Chaque capteur élémentaire permet d'obtenir une variation périodique de l'impédance acoustique. Le décalage entre les variations périodiques respectivement déterminées par chaque capteur élémentaire permet d'estimer la vitesse d'onde de pouls. Ce type de modalité est décrit dans la publication Hermeling E et al. « The dicrotic notch as alternative time-reference point to measure local pulse wave velocity in the carotid artery by means of ultrasonography », Journal of hypertension, 2009, 2028-2035.

Le paramètre physiologique peut également être mesuré par un unique capteur, par exemple un unique photodétecteur. C'est par exemple le cas lorsque le paramètre physiologique mesuré est une amplitude de vibration de l'onde de pouls. Un tel paramètre est mesurable avec un capteur de type PPG.

D'une façon générale, le ou les capteurs sont configurés pour former une impulsion sous l'effet de l'onde de pouls. Le paramètre physiologique est déterminé en fonction d'une aire ou d'une hauteur de l'impulsion (par exemple lors de la mesure de l'amplitude de vibration de l'onde de pouls), soit à partir d'un décalage temporel entre deux impulsions mesurées par deux capteurs espacés l'un de l'autre (par exemple lors de la mesure de la vitesse d'onde de pouls)..

Le dispositif peut également comporter un capteur de pression 4, configuré pour déterminer la pression selon laquelle le dispositif est appuyé contre l'utilisateur. Les données résultant du capteur de pression 4 sont transmises à l'unité de traitement 30.

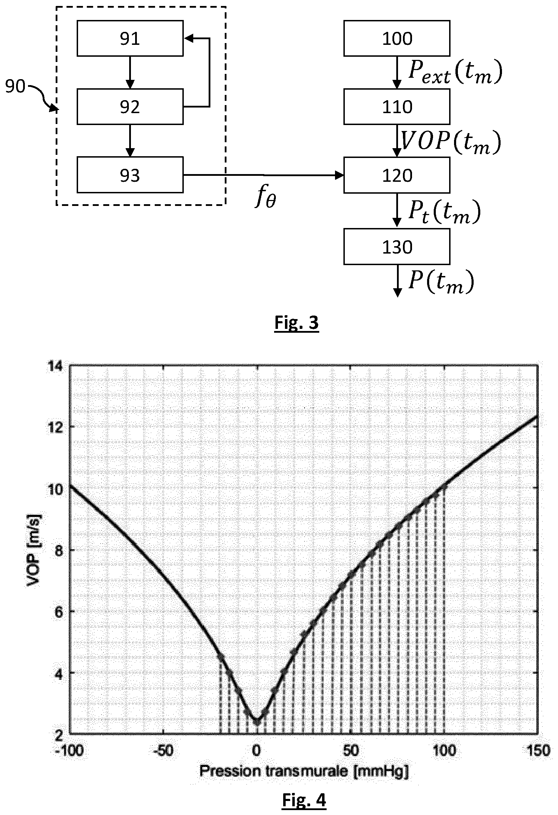

Le dispositif 1 comporte une unité de traitement 30. L'unité de traitement est reliée aux photodétecteurs 21 et 22, de façon à mesurer la vitesse d'onde de pouls. L'unité de traitement 30 est programmée pour mettre en oeuvre les étapes décrites sur la figure 3. L'unité de traitement peut notamment comporter un microprocesseur ou un microcontrôleur.

Les étapes 91 à 93 correspondent à une phase de calibration 90. Au cours de l'étape 91, le brassard 3 est gonflé, de façon à faire varier la pression appliquée sur l'artère. Cela permet de faire varier la pression transmurale. Dans cet exemple, la pression est augmentée progressivement, ce qui entraîne une réduction progressive de la pression transmurale. Au cours de l'étape 92, on mesure la VOP (ou tout autre paramètre physiologique variant sous l'effet d'une variation de la pression transmurale).

Les étapes 91 et 92 sont réitérées, en augmentant (ou réduisant) progressivement la pression appliquée sur l'artère.

La figure 4 schématise une augmentation progressive de la pression appliquée (ou pression externe), qui se traduit par une réduction progressive de la pression transmurale. Chaque ligne verticale correspond à un point de mesure, à un instant de calibration. A chaque instant de calibration, la vitesse d'onde de pouls est mesurée par le dispositif. La diminution progressive de la pression transmurale entraîne une réduction de la vitesse d'onde de pouls, jusqu'à l'atteinte d'une valeur minimale de la vitesse d'onde de pouls. La valeur minimale de la vitesse d'onde de pouls est atteinte lorsque la pression transmurale est nulle, c'est-à-dire lorsque la pression exercée sur l'artère correspond à la pression artérielle moyenne. Lorsque la pression appliquée est augmentée, la pression transmurale est négative et la vitesse d'onde de pouls ré-augmente progressivement. Les itérations des étapes 91 et 92 sont alors arrêtées, car la vitesse d'onde de pouls minimale a été atteinte.

La valeur minimale de la VOP est obtenue en appliquant une pression correspondant à une pression dite de référence. A la pression de référence $P_{ext,ref}$, la pression transmurale de l'artère est considérée comme nulle. La pression de référence correspond à la pression artérielle moyenne de l'utilisateur lors de la phase de calibration.

A l'issue des étapes 91 et 92, on dispose de couples valeurs de VOP et de pression externes mesurées à chaque instant de calibration. Ainsi, si $t_c$ correspond à un instant de calibration, on dispose, à chaque instant de calibration, de couples $(P_{ext}(t_c), VOP(t_c))$ où $P_{ext}(t_c)$ est la pression externe appliquée à un instant de calibration. Le terme pression externe désigne la pression appliquée sur l'artère. On dispose également de la pression $P_{ext,ref}$ de référence, à laquelle la vitesse d'onde de pouls est minimale.

A partir des couples $(P_{ext}(t_c), VOP(t_c))$ et de la valeur de la pression de référence $P_{ref}$, l'étape 93 consiste à déterminer une fonction de calibration, décrivant l'évolution de la pression transmurale $P_t$ en fonction de la vitesse d'onde de pouls.

$$P_t(t_c) = P_{ext,ref} - P_{ext}(t_c) = f_\theta\big(VOP(t_c)\big) \quad (3)$$

La fonction de calibration $f_\theta$, paramétrée par un ensemble de paramètres $\theta$, peut être analytique, par exemple une fonction polynomiale ou autre. Il peut également s'agir d'une fonction déterminée par un réseau de neurones ou, de façon plus générale, un algorithme d'intelligence artificielle à apprentissage supervisé. La fonction de calibration peut être déterminée par l'application d'un modèle de régression aux couples $(P_t(t_c), VOP(t_c))$ résultant des étapes 91 et 92. Il peut par exemple s'agir d'une régression linéaire.

Au cours de la phase de calibration, on suppose que la pression artérielle moyenne est constante. Aussi, la phase de calibration est de préférence réalisée selon un intervalle de temps faible, par exemple quelques dizaines de secondes ou quelques minutes.

Les étapes 100 à 130 sont effectuées au cours d'instants de mesures $t_m$, postérieurement à la phase de calibration. Au cours d'une étape 100, le brassard applique une pression externe $P_{ext}(t_m)$ sur l'artère, la pression externe étant mesurée par le capteur 4.

De préférence, à la différence de l'art antérieur, la pression appliquée est strictement inférieure à la pression de référence $P_{ext,ref}$. Elle est de préférence inférieure à 50% voire de 25% de la pression de référence $P_{ext,ref}$. Elle peut par exemple être égale à 50 mm de mercure. La pression appliquée est supérieure à une pression minimale, déterminée au cas par cas, en fonction du dispositif mis en oeuvre.

Dans les dispositifs de l'art antérieur, mettant en oeuvre un brassard couplé à un capteur de pression, la mesure de la pression artérielle moyenne nécessite un gonflement du brassard, au-delà de l'atteinte du maximum de compliance de l'artère. La pression exercée sur l'artère atteint puis dépasse la pression artérielle moyenne. Si une mesure de la pression artérielle moyenne doit être effectuée de façon répétée, il en résulte un certain inconfort pour l'utilisateur, du fait du niveau de pression appliqué. De plus, cela n'est pas compatible avec une utilisation au cours du sommeil de l'utilisateur. Le fait d'appliquer une pression de mesure inférieure à la pression de référence $P_{ext,ref}$ augmente le confort de l'utilisateur par rapport aux dispositifs de l'art antérieur, dans lesquels la pression appliquée atteint ou dépasse la pression artérielle. Ainsi, la mesure de la pression artérielle peut être effectuée en limitant la gêne pour l'utilisateur.

Au cours de l'étape 110, la VOP, à l'instant de mesure, notée $VOP(t_m)$ est mesurée par le capteur 2.

Au cours de l'étape 120, la pression transmurale est estimée à partir de la fonction de calibration

$$P_t(t_m) = f_\theta(VOP(t_m)) \quad (4)$$

Au cours de l'étape 130, la pression artérielle, à l'instant de mesure, est estimée par l'expression :

$$P(t_m) = P_t(t_m) + P_{ext}(t_m) \quad (5)$$

Les étapes 100 à 130 peuvent être mises en oeuvre en différents instants de mesure.

Les équations (4) et (5) constituent un aspect clef de l'invention. Contrairement à l'art antérieur, la fonction de calibration permet d'estimer, à partir du paramètre physiologique mesuré, non pas la pression artérielle, mais la pression transmurale. Une telle fonction de calibration est bien plus facile à obtenir, puisqu'il suffit de faire varier la pression appliquée sur l'artère et de connaître la pression de référence, pour laquelle la pression transmurale est considérée comme nulle. Contrairement à l'art antérieur, la calibration n'impose pas de faire varier la pression artérielle de l'utilisateur. Au contraire, la pression artérielle est de préférence considérée comme fixe lors de la calibration. Cela est justifié par le fait que la calibration est rapide à effectuer : il suffit de faire varier la pression jusqu'à ou à partir de la pression de référence, pour laquelle le paramètre physiologique atteint une valeur extrémale, maximale ou minimale. Lorsque la valeur extrémale est atteinte, on considère que la pression externe appliquée correspond à la pression artérielle moyenne de l'utilisateur.

A partir de la pression transmurale, estimée en appliquant la fonction de calibration au paramètre physiologique mesuré (équation 4), la pression artérielle est obtenue simplement, par une simple addition de la pression transmurale estimée $P_t(t_m)$ et de la pression externe appliquée $P_{ext}(t_m)$ (équation 5). La pression appliquée est soit mesurée, soit prédéterminée, donc connue.

Outre les paramètres $\theta$ déterminés par la régression, la fonction de calibration peut prendre en compte des paramètres anthropométriques, par exemple le taille, le poids, le sexe ou l'âge.

La pression $P(t_m)$ estimée lors de l'expression (5) correspond à une pression artérielle à l'instant de mesure $t_m$. Lorsque la pression $P_{ext}(t_m)$ est mesurée par un capteur de pression ayant un temps de réponse long, supérieure à plusieurs centaines de ms ou à 1 seconde, la pression $P(t_m)$ correspond à une pression artérielle moyenne sur un ou quelques battements. En effet, compte tenu du faible temps de réponse, la pression $P_{ext}(t_m)$ correspond à une pression moyenne appliquée.

Lorsque la pression $P_{ext}(t_m)$ est mesurée par un capteur de pression ayant un temps de réponse court, par exemple de l'ordre de quelques dizaines de ms, la pression $P(t_m)$ correspond à une pression artérielle « temps réel », à l'instant de mesure $t_m$. La pression $P(t_m)$ prend en compte l'évolution de la pression artérielle entre deux battements consécutifs. On accède ainsi à une composante, dite pulsatile, de la pression artérielle. En fonction des instants de mesure, on peut obtenir la pression systolique (pression maximale au sein d'un même cycle) ou la pression diastolique (pression minimale au sein d'un même cycle). Selon cette possibilité, les instants de mesure sont espacés selon une fréquence supérieure à la fréquence cardiaque de l'utilisateur, par exemple 5 ou 10 fois supérieure. On peut alors estimer l'évolution de la pression transmurale entre deux battements successifs. Cela permet d'obtenir l'évolution de la pression artérielle entre deux battements successifs.

Comme précédemment indiqué, l'invention peut être mise en oeuvre avec un autre paramètre physiologique. Il peut par exemple s'agir de l'amplitude d'oscillation de l'artère. Dans ce cas, au cours de la phase de calibration, on augmente progressivement la pression jusqu'à l'atteinte d'un maximum d'amplitude de vibration. On n'a alors besoin seulement d'un seule couple source-détecteur, par exemple la première source élémentaire 11 et le premier photodétecteur élémentaire 21. Lorsque l'amplitude maximale est atteinte, la pression appliquée correspond à la pression de référence, c'est-à-dire à la pression artérielle moyenne de l'utilisateur durant la phase de calibration.

On trouvera ci-dessous d'autres exemples de paramètres physiologiques exploitables :

- temps de transit de pouls, c'est-à-dire le temps de transit de l'onde de pouls entre deux points de mesure, usuellement désigné par l'acronyme « PWTT » (Puise wave transit time - temps de transit de l'onde de pouls) ;
- durée entre le pic systolique et le pic diastolique d'un même battement ;
- aires du pic systolique ou du pic diastolique tels que mesurés par le détecteur.

Ainsi, d'une façon générale, le capteur est configuré pour obtenir une impulsion sous l'effet d'une onde de pouls : il peut s'agir, à titre non limitatif, d'un capteur optique, acoustique, électrique, électromécanique (accéléromètre).

La phase de calibration peut être renouvelée périodiquement, de façon à pouvoir mettre à jour la fonction de calibration. La mise à jour peut être effectuée régulièrement, par exemple de façon hebdomadaire.

Selon une possibilité, plusieurs paramètres physiologiques peuvent être utilisés pour déterminer la pression artérielle. Chaque paramètre physiologique atteint une valeur extrémale (minimale ou maximale) lorsque la pression transmurale est nulle. Dans ce cas, la fonction de calibration est multiparamétrique, au sens où elle dépend de chaque paramètre physiologique considéré. La calibration est effectuée comme précédemment décrit,

- en augmentant la pression appliquée sur l'artère jusqu'à une pression de référence, à laquelle chaque paramètre est considéré comme ayant atteint sa valeur maximale ou sa valeur minimale ;
- et/ou en diminuant la pression appliquée sur l'artère à partir d'une pression de référence, à laquelle chaque paramètre est considéré comme ayant atteint sa valeur maximale ou sa valeur minimale.

A partir des différentes valeurs de paramètres mesurées pour chaque pression appliquée, et à partir de la pression de référence, pour laquelle la pression transmurale est considérée comme nulle, on détermine la fonction de calibration, par exemple par régression.

L'invention permet une mesure fréquente de la pression artérielle de l'utilisateur, en différents instants d'une même journée en limitant la gêne de l'utilisateur. Cela est particulièrement adapté aux utilisateurs nécessitant un suivi régulier de la pression artérielle.

**Revendications**

1. Procédé de détermination d'une pression artérielle d'un utilisateur, en mesurant un paramètre physiologique, le paramètre physiologique passant par un extremum lorsque la pression transmurale de l'artère est nulle, le procédé comportant les étapes suivantes :

   - a) application d'une pression ($P_{ext}(t_c)$) sur l'artère, de façon à modifier la pression transmurale de l'artère, la pression transmurale correspondant à une différence entre la pression artérielle et la pression appliquée sur l'artère, la pression appliquée étant connue ou mesurée ;
   - b) simultanément à l'étape a), mesure du paramètre physiologique ($VOP(t_c)$) de l'utilisateur à l'aide d'un capteur;

   les étapes a) et b) étant réitérées en différents instants de calibration ($t_c$), en modifiant la pression appliquée sur l'artère, de façon que d'une itération, l'étape b) comporte une application d'une pression de référence, à laquelle, l'extremum du paramètre physiologique est mesuré, la pression appliquée é alors à une pression de référence, qui correspond à la pression artérielle moyenne de l'utilisateur lors des instants de calibration;

   - c) à partir

     • de la pression appliquée et du paramètre physiologique mesuré à chaque instant de calibration ;
     • et de la pression de référence ;

   détermination de la pression transmurale à chaque instant de calibration et établissement d'une fonction de calibration, la fonction de calibration déterminant une relation entre la pression transmurale et le paramètre physiologique;

- d) suite aux instants de calibration, application d'une pression ($P_{ext}(t_m)$) sur l'artère, à au moins un instant de mesure ($t_m$) et mesure du paramètre physiologique à l'instant de mesure ($VOP(t_m)$), la pression appliquée à l'instant de mesure étant connue ou mesurée ;

- e) application de la fonction de calibration résultant de c) au paramètre physiologique mesuré à l'instant de mesure, de façon à estimer une pression transmurale à l'instant de mesure ($P_t(t_m)$);

- f) à partir de la pression transmurale estimée lors de l'étape e) et de la pression appliquée à l'instant de mesure, estimation d'une pression artérielle de l'utilisateur.

2. Procédé selon la revendication 1, dans lequel l'étape f) comporte une addition de la pression transmurale estimée lors de l'étape e) et de la pression appliquée lors de l'étape d).

3. Procédé selon l'une quelconque des revendications précédentes, dans lequel le paramètre physiologique est un paramètre relatif à la compliance de l'artère.

4. Procédé selon l'une quelconque des revendications précédentes, dans lequel lors de l'étape a) ou de l'étape d), la pression est appliquée au moyen d'un moyen d'application d'une pression sur l'artère de l'utilisateur (3), configuré pour comprimer l'artère.

5. Procédé selon l'une quelconque des revendications précédentes, dans lequel la fonction de calibration est obtenue en appliquant un modèle de régression à partir de la pression appliquée et du paramètre physiologique mesuré à chaque instant de calibration.

6. Procédé selon l'une quelconque des revendications précédentes, dans lequel lors de l'étape d), la pression appliquée sur l'artère est inférieure à la pression de référence.

7. Procédé selon la revendication 6, dans lequel lors de l'étape d), la pression appliquée sur l'artère est inférieure à 50% ou à 25 % de la pression de référence.

8. Procédé selon l'une quelconque des revendications précédentes, dans lequel :

- les étapes a) et b) sont mises en oeuvre en prenant en compte différents paramètres physiologiques, de façon à mesurer un extremum de chaque paramètre physiologique ;
- lors de l'étape c), la fonction de calibration détermine une relation entre la pression transmurale et chaque paramètre physiologique ;
- l'étape d) comporte une mesure de chaque paramètre physiologique à l'instant de mesure;
- lors de l'étape e), la pression transmurale à l'instant de mesure est estimée à partir de la fonction de calibration déterminée lors de l'étape c) et des paramètres physiologiques mesurés lors de l'étape d).

9. Procédé selon l'une quelconque des revendications précédentes, dans lequel les étapes d) à f) sont mises en oeuvre en différents instants de mesure, selon une fréquence supérieure à une fréquence cardiaque de l'utilisateur, de façon à obtenir une évolution de la pression artérielle de l'utilisateur entre lesdits instants de mesure.

10. Procédé selon l'une quelconque des revendications 1 à 8, dans lequel lors de l'étape f), la pression artérielle estimée est une pression artérielle moyenne de l'utilisateur.

11. Dispositif (1) d'estimation d'une pression artérielle d'un utilisateur, comportant :

- un capteur (2), configuré pour être appliqué face à une artère de l'utilisateur, et configuré pour mesurer un paramètre physiologique de l'utilisateur, le paramètre physiologique passant par un extremum lorsque la pression transmurale de l'artère est nulle ;
- un moyen (3) d'application d'une pression sur l'artère de l'utilisateur, configuré pour appliquer une pression variable sur l'artère de l'utilisateur ;
- une unité de traitement (30), destinée à mettre en oeuvre les étapes c), e) et f) d'un procédé selon l'une quelconque des revendications précédentes à partir du paramètre physiologique mesuré par le capteur (2) et de la pression appliquée sur l'artère.

**12.** Dispositif selon la revendication 11, comportant un capteur de pression (4), configuré pour quantifier la pression appliquée sur l'artère par le moyen d'application d'une pression sur l'artère de l'utilisateur.

**13.** Dispositif selon la revendication 11 ou la revendication 12, dans lequel le capteur est choisi parmi : un capteur acoustique, un capteur optique, un capteur tonométrique ou un capteur d'impédance, ou un capteur électromécanique.

**14.** Dispositif selon l'une quelconque des revendications 11 à 13, dans lequel le capteur est maintenu autour d'un membre de l'utilisateur par un bracelet, le moyen d'application d'une pression sur l'artère de l'utilisateur étant intégré dans le bracelet ou solidaire du bracelet.

**Fig. 1A**

**Fig. 1B**

**Fig. 2A**

**Fig. 2B**

**Fig. 3**

**Fig. 4**

Europäisches
Patentamt

European
Patent Office

Office européen
des brevets

## RAPPORT DE RECHERCHE EUROPEENNE

Numéro de la demande

**EP 23 18 8547**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2010/241011 A1 (MCCOMBIE DEVIN B [US] ET AL) 23 septembre 2010 (2010-09-23) * alinéas [0049] – [0053], [0067], [0082] – [0084]; figures 2,7 * * le document en entier * | 1-14 | INV. A61B5/0225 A61B5/0285 ADD. A61B5/02 |
| X | US 2017/215749 A1 (ZHUO XIAODING [US] ET AL) 3 août 2017 (2017-08-03) * alinéas [0047] – [0050], [0058] – [0059], [0074] – [0078], [0138] – [0139] * * le document en entier * | 1-14 | |

**DOMAINES TECHNIQUES RECHERCHES (IPC)**

**A61B**

Le présent rapport a été établi pour toutes les revendications

| Lieu de la recherche | Date d'achèvement de la recherche | Examinateur |
|---|---|---|
| **La Haye** | **22 novembre 2023** | **Furlan, Stéphane** |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.....................................................................
& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 23 18 8547

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

22-11-2023

| Document brevet cité au rapport de recherche | Date de publication | Membre(s) de la famille de brevet(s) | Date de publication |
|---|---|---|---|
| US 2010241011 A1 | 23-09-2010 | AUCUN | |
| US 2017215749 A1 | 03-08-2017 | US 2017215749 A1 | 03-08-2017 |
| | | WO 2017136772 A1 | 10-08-2017 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20100241011 A

- US 20170215749 A

**Littérature non-brevet citée dans la description**

- **BASSEM I et al.** *Multi-source multi-frequency bio-impedance measurement method for localized pulse wave monitoring,* 2020

- **HERMELING E et al.** The dicrotic notch as alternative time-reference point to measure local pulse wave velocity in the carotid artery by means of ultrasonography. *Journal of hypertension,* 2009, 2028-2035